# EUROPEAN PATENT APPLICATION

(11) **EP 3 295 933 A1**
(43) Date of publication of application: **21.03.2018**
(21) Application number: 16188771.6
(22) Date of filing: 14.09.2016
(51) Int. Cl.: A61K 9/06, A61K 47/36, A61K 9/50, C08B 37/00, C08L 5/04

(54) **HYDROGELS BASED ON FUNCTIONALIZED POLYSACCHARIDES**

(71) Applicant: ECOLE POLYTECHNIQUE FEDERALE DE LAUSANNE (EPFL), 1015 Lausanne (CH)
(72) Inventor: GERBER, Sandrine, 1163 ETOY (CH); PASSEMARD, Solène, 1022 CHAVANNES-RENENS (CH); WANDREY, Christine, 1024 ECUBLENS (CH); BUHLER, Léo, 1223 COLOGNY (CH); MOREL, Philippe, 1253 VANDOEUVRES (CH)
(74) Representative: KATZAROV S.A.

(57) **Abstract**

The present invention relates to functionalized hydrogel networks grafted with at least one moiety for use in numerous fields, from cosmetics to surgery and medicine.

## Description

### FIELD OF THE INVENTION

The present invention relates to a functionalized hydrogel network grafted with at least one moiety for use in numerous fields, from cosmetics to surgery and medicine.

### BACKGROUND OF THE INVENTION

The increasing incidence of age-related diseases and the limited availability of human donor material to replace dysfunctional cells and damaged tissues in patients have prompted the search for alternative transplantation therapies. Hydrogels, presenting a three dimensional (3D) structure, can serve as scaffolds for tissue engineering and as carrier for cells and drug delivery. The allo-/xeno-transplantation of encapsulated cells/tissues prevents adverse immunological response while allowing the crossing of oxygen, nutrients and secreted factors. In addition, cell microencapsulation offers protection against mechanical stress or deteriorating environmental effects. The water-soluble biopolymer sodium alginate (Na-alg) spontaneously forms hydrogels in the presence of some divalent cations, under mild conditions of pH and temperature [1, 2]. In particular, the hydrogel calcium alginate (Ca-alg) presents favorable properties for several biological and medical applications, including cell immobilization [3]. Despite the highly promising studies that have been directed toward the development of hydrogel microcapsules for the treatment of human diseases, routine clinical application of cell containing microcapsules still remains a challenge. Many requirements are imposed on the microcapsule materials: biocompatibility with both the host tissue and the enclosed cells, long-term stability of the mechanical strength and elasticity, maintenance of a favorable environment for cell survival and metabolic functionality, reproducible manufacturing protocols.

Alginate-based microspheres (MS) prepared by ionic cross-linking with divalent cations present, for some applications, insufficient mechanical properties and stability as well as defects in permselectivity [4-7]. To overcome these issues, many efforts were devoted to the improvement of Ca-alg MS or the replacement of Ca-alg by other materials. Representative developments in this field include the coating of Ca-alg MS with polycations such as poly (L-lysine) [8], poly(L-ornithine) [9] or poly(methylene guanidine) [10] and their derivatives, and the use of other polymers such as chitosan [11], poly(ethylene glycol) (PEG) [12], dextran [13] and hyaluronic acid [14]. However these modifications often led to reduced biocompatibility and implied complex multistep MS formation procedures [15]. Other modifications of polymeric hydrogels involve the incorporation of bioactive molecules to improve the viability and functionality of encapsulated cells, and release therapeutic payloads at the site of transplantation. Small peptide sequences have been coupled to alginate molecules to improve cell spreading and growth in the microcapsules but resulted in high degree of degradation and fibrosis in vivo. [16] Another approach consists in the co-encapsulation of selected drugs and cells of interest within hydrogel microcapsules to reduce adverse fibrotic overgrowth at the site of implantation. In particular, the co-encapsulation of rat islets and curcumin in alginate hydrogels resulted in improved glycemic control and reduced pericapsular overgrowth in vivo. [17] An alternative promising approach proposes the design of hydrogel MS combining the fast ionotropic gelation of alginate with covalent cross-linking resulting from PEG derivatives, either covalently linked to the alginate backbone or in the form of an interpenetrating network. Following this concept, we have recently developed two types of two-component MS, composed of Ca-alg and interpenetrating covalently cross-linked networks formed from vinyl sulfone-terminated multiarm PEG [18-20] or Na-alg functionalized with cysteamine [21], which showed favorable properties for cell microencapsulation. In addition, one-component MS resulting from ionotropic gelation and simultaneous covalent cross-linking interactions of PEG-grafted alginates were produced and demonstrated improved mechanical properties and permeability compared to pure Ca-alg MS [21]. Nevertheless, lack of in vivo stability of the MS in immunocompetent mice models asked for the design of new grafting methodologies for the conjugation of thiol-terminated PEG derivatives to the hydroxyl functionalities of the alginate backbone.

### LIST OF ABBREVIATIONS

Alg, alginate; Na-Alg, sodium alginate; Ca-Alg, calcium alginate; Boc, tert-butyloxycarbonyl; CDI, carbodiimidazole; DMF, N,N-dimethyl formamide; DMSO, dimethyl sulfoxide; EDCI, 1-ethyl-3-[3-(dimethylamino)propyl]carbodiimide; FITC, fluorescein isothiocyanate; hr, hour; MOPS, 3-(N-morpholino) ethanesulfonic acid; MS, microsphere; MWCO, molecular weight cut-off; NHS, N-hydroxysuccinimide; PE, petroleum ether; PEG, poly(ethylene glycol); rt, room temperature; TBA, tert-butyl ammonium; TCEP, tris(2-carboxyethyl)phosphine; Ts, tosylate;

### SUMMARY OF THE INVENTION

The present invention relates to functionalized hydrogel networks based on polysaccharide grafted with at least one moiety wherein the hydrogel is an anionic polysaccharide and the moiety is grafted on at least one hydroxyl group of said anionic polysaccharide.

Another aspect of the present invention concerns a composition comprising a functionalized hydrogel of the invention combined with at least one second element selected among cells, proteins, nucleic acids or other molecules.

A further aspect of the invention concerns a pharmaceutical composition comprising a composition of the invention and a pharmaceutically acceptable carrier.

Another aspect concerns a process for preparing a functionalized hydrogel network, the process comprising reacting an anionic polysaccharide with a moiety functionalized to allow the formation of a covalent bond between the polysaccharide and the moiety on one hydroxyl group of the polysaccharide.

Another aspect concerns a process for preparing a functionalized alginate based hydrogel, the process comprising
i) converting one or more carboxylic acid functional groups of alginate or one or more carboxylate functional groups of alginate salt into TBA carboxylates,
ii) modifying one or more hydroxyl groups of said alginate, or salt thereof, by reacting one or more hydroxyl groups in the presence of carbodiimidazole followed by precipitation,
iii) preparing thiol-functionalized PEG derivatives (PEG I, PEG II and PEG III) by
   iii1) reacting the intermediates α-amino-ω-azido poly (ethylene glycol) with protected 3-mercaptopropanoïc acid, followed by simultaneous reduction of the azido group and deprotection of the thiol functionality to produce PEG I, or,
   iii2) conjugating intermediates α-amino-ω-azido poly (ethylene glycol) to activated lipoic acid followed by reduction in the presence of LiAlH₄ so as to deliver PEG II as mixtures of opened (reduced) and closed (oxidized) forms of the lipoyl functionality to produce PEG II,
      or
   iii3) contacting intermediates α-amino-ω-azido poly (ethylene glycol) 1 a and 1 b with a triazole moiety using a click reaction catalyzed by copper species to produce PEG III,
iv) grafting the modified alginate with PEG I, PEG II and/or PEG III to allow the formation of a covalent bond between the functionalized alginate and PEG I, PEG II and/or PEG III on one or more hydroxyl groups of said alginate or salt thereof,
v) purifying alginate grafted to PEG through dialysis and freeze drying.

Another aspect concerns a method of treating a disease or disorder in a human or animal patient, comprising: implanting or transplanting into a human or animal patient a material selected among cells, proteins, nucleic acids or other molecules immobilized or microencapsulated in a functionalized hydrogel of the invention.

A further aspect concerns heterobifunctional PEG, or derivatives thereof, of formula I, II and/or III: 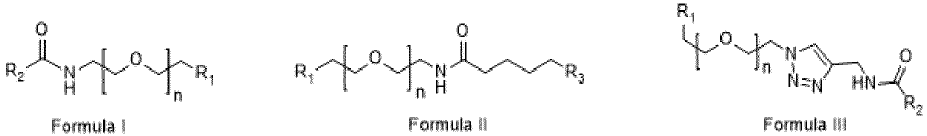 wherein
n = 8 to 50;
R₁ is independently selected from NH₂, N₃, OH, C₁₋₆ alkyl-CO₂H
R₂ is independently selected from (CH₂)ₚSH, with p= 2 to 10, 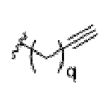 with q = 2 to 5, and 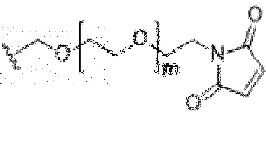 with m= 2 to 10;
R₃ is independently selected from

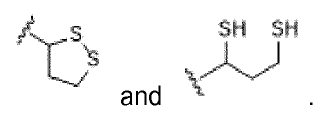

### BRIEF DESCRIPTION OF THE FIGURES

**Figure 1****.** Mechanical resistance to uniaxial compression to 90% of the initial MS diameter. All MS were prepared with PEG grafted alginates resulting from TBA-Alg(x).
**Figure 2****.** Resistance of the MS to 10 successive compressions. A: Comparison of Alg-PEG-a I (3 wt%) and Alg-PEG-b I (4 wt%); B: Comparison of Alg-PEG-a II (3 wt%) and Alg-PEG-b II (4 wt%); C: Comparison of Alg-PEG-a I (3 wt%) and Alg-PEG-a II (3 wt%) and Alg-PEG-a III (3 wt%).; D: Comparison of Alg-PEG-a I (3 wt%) from TBA-Alg(x) and Alg-PEG-a I (2 wt%) from TBA-Alg(y).
**Figure 3****.** Permselectivity of Alg-PEG-a I(x) MS, assessed by ingress diffusion of 40 kDa and 150 kDa FITC-dextran. Fluorescence of the supernatant.
**Figure 4****.** MIN6 cells microencapsulated in MS from Alg-PEG-a I and Alg-PEG-a II (average microsphere diameter: 500µm): photographs are from days 3 and 10 after microencapsulation and culture. Upper panels, light microscopy; middle panel, staining of live cells with fluorescein diacetate; lower panel, staining of dead cells with propidium iodide. For MS from AIG-PEG-a II, the decomposition of the capsular material over time is illustrated in the panel at the bottom.
**Figure 5****.** Glucose-stimulated insulin release for non-encapsulated MIN6 cells and MIN6 cells microencapsulated in MS from Alg-PEG-a I and Alg-PEG-a II.
**Figure 6****.** Primary porcine hepatocytes microencapsulated in MS from Alg-PEG-a I (microsphere diameter about 500µm): photographs are from the day of microencapsulation till day 6 after subsequent culture. Upper panels, light microscopy; middle panel, staining of live cells with fluorescein diacetate; lower panel, staining of dead cells with propidium iodide.
**Figure 7****.** Albumin synthesis of primary porcine hepatocyte in MS from Alg-PEG-a I. The daily synthesis was assessed upon daily exchange of the medium.
**Figure 8****.** Empty MS from Alg-PEG-a I and Alg-PEG-a II, directly after manufacture (microscopy, left panels), and at 30 days after intraperitoneal implantation in mice. Some MS are indicated by arrows.

### DESCRIPTION OF THE INVENTION

The methodology herein reported aims at the production of functionalized hydrogel networks grafted with at least one moiety, in the form of microspheres for different applications including cell therapy, addressing the following issues: tunable in vivo stability (i.e. mechanical, physical and chemical integrity), minimal side effects after transplantation, host and cell acceptance, tunable permeability and retrievalability of the MS.

It must be noted that, as used in this specification and the appended claims, the singular forms "a," "an" and "the" include plural referents unless the content clearly dictates otherwise.

The present invention relates to a functionalized hydrogel grafted with at least one moiety, wherein the polymer is an anionic polysaccharide and the moiety is grafted on at least one hydroxyl group of said anionic polysaccharide.
Preferably, the polysaccharide is an anionic polysaccharide selected from the group comprising dextran, alginate, hyaluronan and galacturonan, a derivative of said polysaccharide or a salt thereof. It can be natural, synthetic or modified. Also envisioned is a combination of two or more anionic polysaccharides, e.g. alginate and hyaluronan.
Preferred anionic polysaccharide derivatives or modified anionic polysaccharide are polysaccharide ethers and polysaccharide esters. They can have one or more substituents, preferably of the types: hydroxyethyl, hydroxypropyl, hydroxybutyl, methyl, ethyl, propyl, dihydroxypropyl, carboxymethyl, sulfoethyl, hydrophobic long-chain branched and unbranched alkyl groups, hydrophobic long-chain branched and unbranched alkyl aryl groups or aryl alkyl groups, acetate, propionate, butyrate, lactate, nitrate or sulfate, of which some groups, such as, for example, hydroxyethyl, hydroxypropyl, hydroxybutyl, dihydroxypropyl and lactate, are capable of forming grafts. Alternatively, the anionic polysaccharide derivatives or modified anionic polysaccharides can be selected among those described in WO2012167223 which is incorporated herein by reference in its entirety. The substituents of the polysaccharides according to the invention are not limited to these groups.
The preferred salts of anionic polysaccharides according to the invention, are selected among the mono- or di-valent salts such as sodium, potassium, magnesium, calcium, strontium, barium, manganese salts. Sodium salts are most particularly preferred.
Most preferably, the anionic polysaccharide is alginate, a derivative thereof, or a salt thereof. Even more preferably, the anionic polysaccharide is sodium-alginate (Na-alginate or Na-alg).

The presence of the hydroxyl groups allows the polysaccharide to interact with the aqueous environment and to participate in hydrogen bonding, both within and between chains. In accordance with the present invention, a moiety is grafted on at least one hydroxyl group of said anionic polysaccharide.
According to the present invention, "grafted" means a covalent link between the anionic polysaccharide and the moiety. Preferably, the moiety is grafted to the anionic polysaccharide through an amide bond or an ether bond.
Alternatively, the moiety is grafted to the anionic polysaccharide through a spacer moiety between the anionic polysaccharide and the moiety. The spacer moiety may be selected among alkylenyl groups, cycloalkylenyl groups, alkenylenyl groups, eventually substituted by one or more substituents. Both the anionic polysaccharide and the moiety may be linked to the spacer on the same carbon atom, or on different carbon atoms.
For the requirements of the present invention, and as indicated herein in the description and claims, the term "functionalized hydrogel" or "functionalized hydrogel network" is understood to mean the necessary presence on the anionic polysaccharide of at least one carboxylate group or carboxylic acid functional group per saccharide monomeric unit. Preferably, there are 1 to 40, more preferably 5 to 40, most preferably 5 to 30 mol% modified hydroxyl groups on the polysaccharide backbone.

The moiety is preferably grafted on one hydroxyl group of said anionic polysaccharide preferably through a carbamate bond or a carbonate bond.

The moiety can be any molecule or compound. Usually, the moiety is selected from the group comprising a Polyethylene Glycol (PEG), or a derivative thereof, and a therapeutic molecule.

In case the moiety is a PEG, or a derivative thereof, then said PEG, or derivative thereof, is a heterobifunctional PEG of formula I, II and/or III. 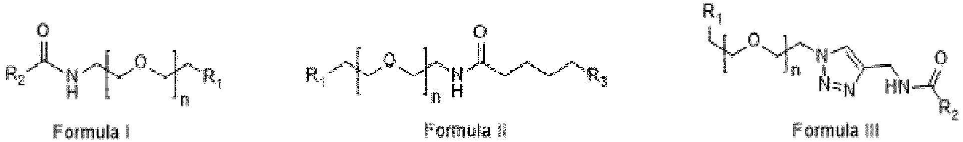 wherein
n = 8 to 50, more preferably n= 15 to 45, even more preferably n= 20 to 44 and even more preferably n= 22, 23, 24, 25, 26, 27, 28, 29, 30, 31, 32, 33, 34, 35, 36, 37, 38, 38, 39, 40, 41, 42, 43 or 44;
R₁ is independently selected from NH₂, N₃, OH, C₁₋₆ alkyl-CO₂H ;
R₂ is independently selected from
   (CH₂)ₚSH, with p= 2 to 10, 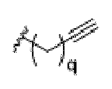 with q = 2 to 5, 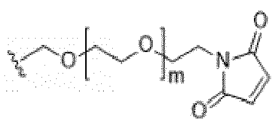 with m= 2 to 10, preferably m= 2 to 8, even more preferably m= 3 to 8 and even more preferably n= 3, 4, 5, 6, 7, or 8;
R₃ is independently selected from 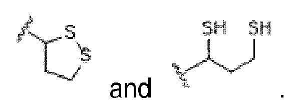
Preferably, the C₁₋₆ alkyl-CO₂H is CH₂CO₂H.

In accordance with the present invention, the PEG, or derivative thereof, is a heterobifunctional PEG derived from linear poly(ethylene glycol) HOCH₂(CH₂OCH₂)ₙCH₂OH with an average value of about 22 (PEG-a) or about 44 (PEG-b). Preferably, the heterobifunctional PEG is selected from the group comprising 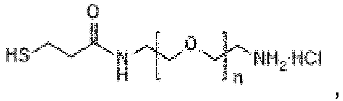 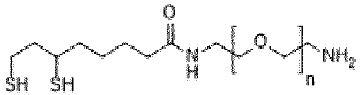 and 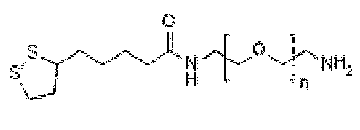 wherein n is comprised between 10 to 60, preferably between 20 to 50, and more preferably between 22 and 44.

The functionalized hydrogel of the invention is preferably in the form of a microsphere. Microspheres (MS) can be obtained by any techniques known in the art, such as for example spray-drying, emulsification-precipitation, water-in-oil emulsion solvent diffusion, emulsification-cross-linking methods and upon droplet extrusion into a gelation bath.

For example, microspheres of a functionalized hydrogel such Na-Alg-PEG polymers are prepared by dissolving said Na-Alg-PEG polymers in a solution of 0.4 % NaCl in 100 mM MOPS buffer, pH 7.4 at the desired concentration. After complete dissolution, the polymer solution is directly extruded for gelification into a gelation bath (e.g. 100 mM CaCl₂ in 100 mM MOPS buffer, pH 7.4) containing tween 80 (1/10 000). Microspheres are produced employing a coaxial air-flow droplet generator (such as for e.g. Encapsulator B-395 Pro, Büchi Labortechnik AG, Flawil, Switzerland). The polymer solution, with or without cells, is then extruded into the tenfold volume of the gelation bath containing CaCl₂ 2H₂O (100 mM) in MOPS. The microspheres are separated by filtration, washed twice with CaCl₂ stock solution, and finally stored in this solution at 4 °C, or in cell culture medium in case of cell microencapsulation.

One aspect of the invention also concerns a composition comprising a functionalized hydrogel of the invention combined with at least a second element selected among cells, proteins, nucleic acids or other molecules. Preferably, the second element is encapsulated within the microspheres formed from the functionalized polymer by directly dispersing said second element in the polysaccharide solution before encapsulation.

The functionalized hydrogels of the invention can be used in numerous fields, from cosmetics to surgery and medicine. For example, they can be used, alone or combined with at least one second element, as films and membranes in various sectors of medicine, such as ophthalmology, dermatology, otorhinolaryngology, neurology, internal and cardiovascular surgery in particular as tissue substitutes and as agents to enable the adhesion of tissue surfaces (such as severed nerves) or in preventing surgical adherence, or for wound dressings.

The functionalized hydrogels of the invention can also be advantageously combined with cells from human, animal or vegetal origin, such as, e.g., keratinocytes, fibroblasts, osteocytes, chondrocytes, urocytes, stem cells, endothelial cells, pancreatic cells, liver cells and neural cells. Encapsulated cells can be transplanted into a patient in need thereof to treat a disease or disorder. In some aspects, the encapsulated cells are obtained from a genetically non-identical member of the same species or from genetically modified cells. In alternative aspects, the encapsulated cells are obtained from a different species than the patient. In preferred aspects, hormone- or protein-secreting cells are encapsulated and transplanted into a patient to treat a disease or disorder.

Moreover as above explained they can be advantageously utilized as coating for organs, such as cardiac valves, or blood vessels, or of biomedical articles such as urologic catheters. This type of coating improves to a high degree the biocompatibility of the article to be grafted, thereby improving the performance thereof at a biological level.

In particular the functionalized hydrogels of the invention can be used as coating for blood vessels following coronary angioplasty, in repair following the dissection of blood vessels and the attachment of flaps on the walls of the same, following spontaneous detachment or lesion, and in the sealing of aneurisms.

In some aspects of the invention described herein, the functionalized hydrogel of the invention or a pharmaceutically acceptable salt thereof, is present in a pharmaceutical composition, along with at least one pharmaceutically acceptable carrier. Pharmaceutically acceptable carriers may include pharmaceutically adjuvants and/or other excipients, and other ingredients can be deemed pharmaceutically acceptable insofar as they are compatible with other ingredients of the formulation and not deleterious to the recipient thereof. The aim of the pharmaceutical composition of the invention is thus to provide functionalized hydrogels presenting the required properties for the stabilization, administration and delivery of a great diversity of active principles.
Depending on the condition being treated, the pharmaceutical composition can be formulated and administered systemically or locally. In one aspect of the present invention is targeted at providing functionalized polysaccharides, intended for the stabilization, administration and delivery of active principles, which can be prepared by methods which are relatively simple to employ and which offer an increased ability to be adjusted in terms of interaction properties. The aim of this aspect of the present invention is thus to provide functionalized polysaccharides presenting the required properties for the stabilization, administration and delivery of a great diversity of active principles.
For example, the pharmaceutical composition reported herein can serve as carrier for the controlled delivery of molecular cargos (drugs, peptide sequences) after transplantation. Several anti-inflammatory agents (for instance, curcumin and ketoprofen) were derivatized for further conjugation to Alg-PEG microspheres, either by coupling to alginate hydroxyl moieties or covalent linking to the grafted PEG chains as shown below:

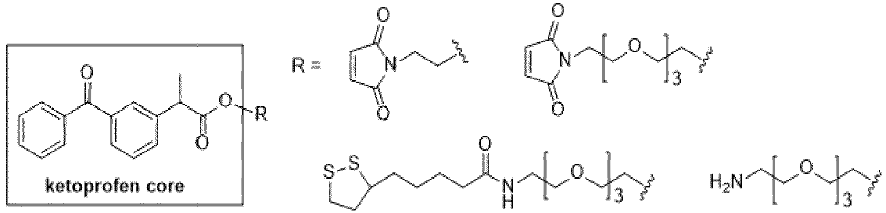

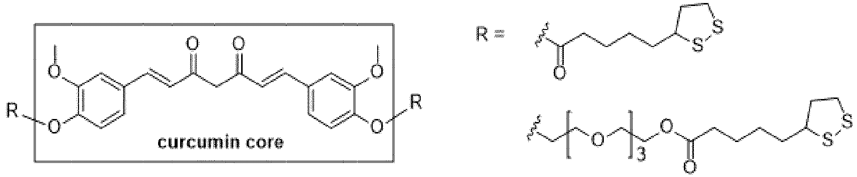

Provided herein are also methods and processes for preparing functionalized hydrogels of the invention.
Typically, a process for preparing a functionalized hydrogel, comprises reacting an anionic polysaccharide with a moiety functionalized to allow the formation of a covalent bond between the polysaccharide and the moiety on at least one hydroxyl group of the polysaccharide.
Preferably, the polysaccharide is an anionic polysaccharide that comprises one or more carboxylic acid functional groups. This anionic polysaccharide is preferably selected from the group comprising dextran, alginate, hyaluronan and galacturonan, a derivative of said polysaccharide or a salt thereof. It can be natural, synthetic or modified. Also envisioned is a combination of two or more anionic polysaccharides, e.g. alginate and hyaluronan.
Usually, the moiety is selected from the group comprising a Polyethylene Glycol (PEG), or a derivative thereof, and a therapeutic molecule.

In case the moiety is a PEG, or a derivative thereof, then said PEG or a derivative thereof, is a heterobifunctional PEG of formula I, II and/or III. 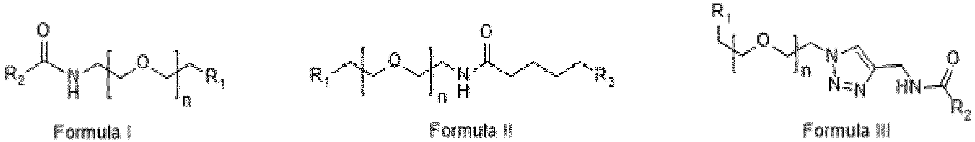 wherein
n = 8 to 50; more preferably n= 15 to 45, even more preferably n= 20 to 44 and even more preferably n= 22, 23, 24, 25, 26, 27, 28, 29, 30, 31, 32, 33, 34, 35, 36, 37, 38, 38, 39, 40, 41, 42, 43 or 44;
R₁ is independently selected from NH₂, N₃, OH, C₁₋₆ alkyl-CO₂H;
R₂ is independently selected from:
   (CH₂)ₚSH, with p= 2 to 10, 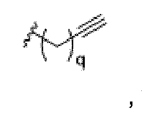 with q = 2 to 5 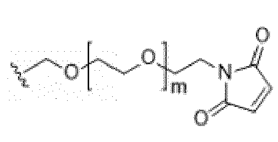 with m= 2 to 10, more preferably m= 2 to 8, even more preferably m= 3 to 8 and even more preferably n= 3, 4, 5, 6, 7, or 8;
R₃ is independently selected from 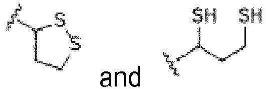
Preferably, the C₁₋₆ alkyl-CO₂H is CH₂CO₂H.

In accordance with the present invention, the PEG, or derivative thereof, is a heterobifunctional PEG derived from linear poly(ethylene glycol) HOCH₂(CH₂OCH₂)ₙCH₂OH with average n value of 22 (PEG-a) or 44 (PEG-b). Preferably, the heterobifunctional PEG is selected from the group comprising 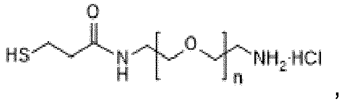 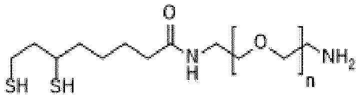 and 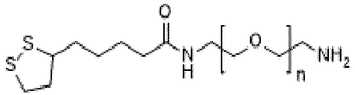 wherein n is comprised between 22 and 44.

A particular aspect of the present invention concerns a process for preparing a functionalized alginate based hydrogel, the process comprising
i) converting one or more carboxylic acid functional groups of alginate or one or more carboxylate functional groups of alginate salt into TBA carboxylates,
ii) modifying one or more hydroxyl groups of said alginate, or salt thereof, by reacting one or more hydroxyl groups in the presence of carbodiimidazole followed by precipitation,
iii) preparing thiol-functionalized PEG derivatives (PEG I, PEG II and PEG III) by
   iii1) reacting the intermediates α-amino-ω-azido poly (ethylene glycol) with protected 3-mercaptopropanoïc acid, followed by simultaneous reduction of the azido group and deprotection of the thiol functionality,
      or,
   iii2) conjugating intermediates α-amino-ω-azido poly (ethylene glycol) to activated lipoic acid followed by reduction in the presence of LiAlH₄ so as to deliver PEG II as mixtures of opened (reduced) and closed (oxidized) forms of the lipoyl functionality,
      or
   iii3) contacting intermediates α-amino-ω-azido poly (ethylene glycol) 1 a and 1 b with a triazole moiety using a click reaction catalyzed by copper species to produce PEG III,
iv) grafting the functionalized alginate with PEG I, PEG II and/or PEG III to allow the formation of a covalent bond between the functionalized alginate and PEG I, PEG II and/or PEG III on one hydroxyl group of said alginate or salt thereof, and v) purifying the PEG-grafted alginate through dialysis and freeze drying.

The above process for preparing a functionalized alginate-based hydrogel was presented for the functionalization of the biopolymer sodium alginate with thiol containing poly(ethylene glycol) units. The first step of the synthetic pathway, which consists in heterogeneous acidification of Na-alg, can be adjusted to provide intermediates TBA-alg of different molar mass. From these intermediates, Alg-PEG derivatives are obtained by activation of hydroxyl moieties in the form of imidazolide, followed by formation of covalent carbamate linkage with heterocheletic PEG derivatives. TBA-alg intermediate of higher molecular mass led to more viscous solutions of the resulting Alg-PEG systems. The combination of ionic gelation with calcium ions and covalent self-cross-linking of the thiol moieties leads to MS presenting mechanical properties and permselectivity suitable for cell transplantation applications. As shown in the examples, fine-tuning of the MS properties is obtained by variation on the PEG length and functionalities, degree of grafting and alg-PEG concentration. In addition, alginate chain degradation can be forced or avoided by slight modification of the grafting protocol.

The present invention also envisions a method of treating and/or preventing a disease or disorder in a human or animal patient, comprising: implanting or transplanting into a human or animal patient a material selected among cells, proteins, nucleic acids or other molecules encapsulated in a functionalized hydrogel of the invention.

*In vitro* and *in vivo* studies demonstrated the compatibility of functionalized hydrogels of the invention for cell microencapsulation and their potential for cell transplantation. Depending on the targeted applications, the properties of these functionalized hydrogel can be tuned to achieve long-term *in vivo* durability or degradation over time.

Also envisioned is a pharmaceutical composition of the invention for use in treating and/or preventing a disease or disorder in a human or animal patient.

Another aspect also concerns the use of a functionalized hydrogel of the invention in the preparation of a medicament or drug or therapeutic product for the treatment and/or prevention of a disease or disorder in a human or animal patient

The present invention also encompasses heterobifunctional PEG oligomers suitable for orthogonal functionalization on both ends of the polymers, or a derivative thereof, of formula I, II and/or III: 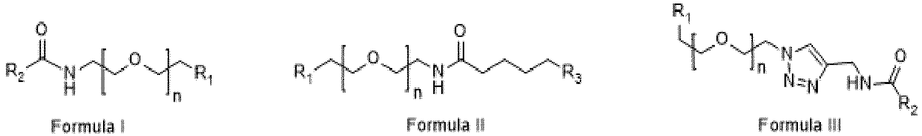 wherein
n = 8 to 50; more preferably n= 15 to 45, even more preferably n= 20 to 44 and even more preferably n= 22, 23, 24, 25, 26, 27, 28, 29, 30, 31, 32, 33, 34, 35, 36, 37, 38, 38, 39, 40, 41, 42, 43 or 44;
R₁ is independently selected from NH₂, N₃, OH, C₁₋₆ alkyl-CO₂H;
R₂ is independently selected from (CH₂)ₚSH, with p= 2 to 10, 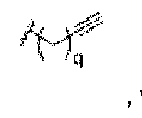 with q = 2 to 5, 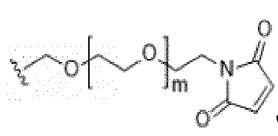 with m= 2 to 10, more preferably m= 2 to 8, even more preferably m= 3 to 8 and even more preferably n= 3, 4, 5, 6, 7, or 8;
R₃ is independently selected from 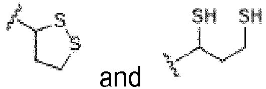
Preferably, the C₁₋₆ alkyl-CO₂H is CH₂CO₂H.

In accordance with the present invention, the PEG, or derivative thereof, is a heterobifunctional PEG derived from linear poly(ethylene glycol) HOCH₂(CH₂OCH₂)ₙCH₂OH with average n value of 22 (PEG-a) or 44 (PEG-b). Preferably, the heterobifunctional PEG is selected from the group comprising 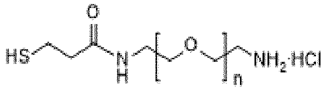 and 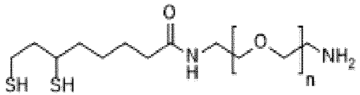 and 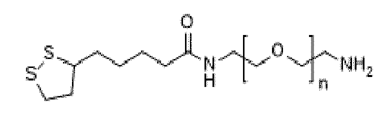 wherein n is comprised between 22 and 44.

### EXAMPLES

### Material & Methods

### Experimental section

### Chemical syntheses - General conditions

Na-alg Kelton HV (lot no. 61650A, [η] = 813 mL g⁻¹ in 0.1 M NaCl, T = 25 °C, G/M = 0.6) was obtained from Kelco (San Diego, USA, CA). Linear PEGs (MM= 1000 g.mol⁻¹ or 2000 g.mol⁻¹) were obtained from Sigma (Buchs, Switzerland). Other commercial reagents (Fluka, Sigma, Switzerland; TCI Europe, Zwijndrecht, Belgium) were used without further purification. Unless special mention, all reactions were performed under argon atmosphere (1 atm). Anhydrous solvents were obtained by filtration (Innovative Technology, Oldham, UK). Reactions were monitored by TLC (Merck silica gel 60F254 plates, Merck, Darmstadt, Germany). Detection was performed by UV light, KMnO₄, Ninhydrin or I₂. Purifications were performed by flash chromatography on silica gel (Merck N° 9385 silica gel 60, 240-400 mesh). IR spectra were recorded on a Perkin-Elmer-1420 spectrometer (Perkin-Elmer, Waltham, MA, USA). ¹H-NMR spectra were recorded on a Bruker ARX-400 spectrometer (400 MHz) (Bruker, Billerica, MA, USA). ¹³C-NMR spectra were recorded on a Bruker ARX-400 spectrometer (100.6 MHz). Chemical shifts are expressed in parts per million (ppm) and coupling constants (J) in hertz. Solvents used for NMR spectroscopy are deuterated chloroform (CDCl₃, Acros) and deuterated methanol (CD₃OD, Acros). Mass spectra were obtained on a Nermag R-10-10C spectrometer with chemical ionization (NH₃) and mode m/z (amu) [% relative base peak (100%)] (Nermag, Santa Clara, CA, USA).

### Formation of one-component microspheres

Solutions of Na-Alg-PEG polymers were prepared according to the following formulation: Na-alg-PEG was dissolved in a solution of 0.4 % NaCl in 100 mM MOPS buffer, pH 7.4 at the desired concentration. After full dissolution, the polymer solution was directly extruded for gelification into a gelation bath (100 mM CaCl₂ in 100 mM MOPS buffer, pH 7.4) containing tween 80 (1/10 000). MS were produced employing a coaxial air-flow droplet generator (Encapsulator B-395 Pro, Büchi Labortechnik AG, Flawil, Switzerland). In case the polymer is used with cells, the polymer solution is sterilized by filtration. The polymer solution, with or without cells, was extruded into the tenfold volume of the gelation bath containing CaCl₂2H₂O (100 mM) in MOPS. The MS were collected by filtration, washed twice with CaCl₂ stock solution, and finally stored in this solution at 4 °C, or in cell culture medium in case of cell microencapsulation.

### Physical characterization of microspheres

The average diameter was measured on an Olympus AX70 microscope equipped with an Olympus DP70 color digital camera. The mechanical resistance to 90% compression of the initial MS diameter was analyzed using a texture analyzer (TA-XT2i, software Texture Exponent 32, Stable Micro Systems, Godalming, UK) equipped with a force transducer (1 mN resolution). A single MS was placed below the probe, for which a constant speed was set as 0.5 mm s⁻¹. Thirty MS of each batch were included in the analysis. The permeability of the MS was studied by measuring the ingress diffusion of FITC-dextran standards (40, 150 kg mol⁻¹). Prior to the measurement, the MS were equilibrated in the gelation bath. 700 mg of MS, collected by filtration and gently dried on paper, were incubated in 1 mL of FITC-dextran solution (1 mg mL⁻¹ in 100 mM CaCl₂ solution). 40 µL of the supernatant were withdrawn after 1 min (t = 0) and at defined time intervals, and diluted in 600 µL of the gelation bath. Fluorescence spectroscopy (Multiplate Reader Safire-II Tecan, Maennedorf, Switzerland) was used to monitor the FITC-dextran concentration.

### Preparation of compound 2 (Scheme 1)

To a solution of 3-mercaptopropionic acid (1 equiv, 94.2 mmol, 10 g) in H₂O: THF (1:1, 180 mL) were added Boc₂O (1.2 equiv, 113.04 mmol, 24.7 g) and Et₃N (2 equiv, 188.4 mmol, 19.07 g, 25.43 mL) and the reaction mixture was stirred for 12 hr at rt. THF was evaporated under vacuo and 1 M HCl (50 mL) was added. The product was extracted with DCM (3 x 100 mL). The combined organic layers were dried (MgSO₄) and concentrated in vacuo. The product was purified by FCC on silica gel (PE/EtOAc 8:1) to afford **2** as a transparent oil (43. 3 mmol, 8.92 g, 46 %). **¹H-NMR (400 MHz, CDCl₃):** *δ* 11.01 (s, 1 H, COOH), 3.01 (t, *J* = 7.0 Hz, 2H, C*H*₂-S), 2.74(t, *J* = 7.0 Hz, 2H, C*H*₂-COOH), 1.48 (s, 9H, 3 x C*H*₃).
**¹³C NMR (100 MHz, CDCl₃):** *δ* 178.2 (COOH), 168.9 (CO), 85.3 (Cq), 34.8 (*C*H₂), 28.3 (*C*H₃), 25.7 (*C*H₂).
**IR (neat):** 1710, 1370, 1245, 1205, 1125, 825 cm⁻¹.
**HRMS-ESI:** calcd. for C₈H₁₄O₄S: 205.0535; found: 205.0535.

### Preparation of compound 3a (Scheme 1)

EDCI (3 equiv, 5.64 mmol, 875.6 mg) and Et₃N (3 equiv, 5.64 mmol, 570.7 mg) were added to a solution of **1a** (1 equiv, 1.88 mmol, 2 g) and **2** (2 equiv, 3.77 mmol, 778 mg) in DCM (20 mL). The reaction mixture was stirred 24 hr at rt and subsequently washed with sat. NH₄Cl solution (2 x 20 mL) and with sat. NaHCO₃ solution (2 x 20 mL). The organic phase was dried (MgSO₄), concentrated in vacuo. The product was purified by FCC on silica gel (DCM/MeOH 20:1 to 10:1) to afford **3a** as a yellowish oil (1.74 mmol, 1.94 g, 93 %).
**¹H-NMR (400 MHz, CDCl₃):** *δ* 6.30 (s, 1 H,N*H*), 3.69-3.63 (m, 80 H, 40 x C*H*₂-O-CH₂), 3.60-3.54 (m, 2H, C*H*₂-O), 3.47 (t, *J* = 5.2 Hz, 2H, C*H*₂-NH), 3.42-3.38 (m, 2H, C*H*₂-N₃), 3.05 (t, *J* = 7.1 Hz, 2H, C*H*₂-CO), 2.53 (t, *J* = 7.2 Hz, 2H, C*H*₂-S), 1.48 (s, 9H, 3 x C*H*₃).
**¹³C NMR (100 MHz, CDCl₃):** *δ* 170.8 (CO), 169.2 (CO), 84.9 (Cq), 70.7 (*C*H₂), 70.7 (*C*H₂), 70.6 (*C*H₂), 70.6 (*C*H₂), 70.2 (*C*H₂), 70.0 (*C*H₂), 69.8 (*C*H₂), 50.8 (*C*H₂), 39.3 (*C*H₂), 36.6 (*C*H₂), 28.2 (3 x *C*H₃), 26.7 (*C*H₂).
**IR (neat):** 2860, 2110, 1700, 1670, 1545, 1465, 1345, 1290, 1250, 1205, 1110, 1030, 945, 840 cm⁻¹.
**HRMS-ESI:** calcd. for C₅₂H₁₀₂N₄O₂₄SNa: 1221.6503; found: 1221.6501.

### Preparation of PEG-a I (Scheme 1)

To a solution of compound **3a** (1 equiv, 1.46 mmol, 1.5 g) in toluene (7.5 mL) was added PPh₃ (1.8 equiv, 2.70 mmol, 708 mg) and the reaction was stirred for 30 min at rt. Then, 1 M HCl (19.7 mL) was added and the reaction was stirred for 48 hr at rt. The two phases were separated and the organic phase was washed with 1 M HCl (2 x 20 mL). The combined aqueous layers were washed with DCM (10 mL) before being concentrated in vacuo. After co-evaporation with toluene (3 x 10 mL), pure compound **PEG-a I** was obtained as a yellowish amorphous solid (3.37 µmol, 3.32 g, 95 %).
**¹H**-**NMR (400 MHz, CDCl₃):** 7.64 (s, 1 H, N*H*₂), 3.78 (m, 2H, C*H*₂-CH₂-NH₂), 3.63-3.54 (m, 80H, CH₂-O-CH₂), 3.41 (m, 2H, C*H*₂-N₃), 3.10 (m, 2H, C*H*₂-NH₂), 2.78 (m, 2 H, C*H*₂-SH), 2.64 (m, 2 H, C*H*₂-CO), 1.68 (s, 1 H, S*H*).
**¹³C NMR (100 MHz, CDCl₃):** *δ* 172.3 (CO), 70.3 (*C*H₂), 70.2 (*C*H₂), 70.2 (*C*H₂), 70.1 (*C*H₂), 70.0 (*C*H₂), 69.8 (*C*H₂), 69.1 (*C*H₂), 66.7 (*C*H₂), 40.1 (*C*H₂), 39.6 (*C*H₂), 39.2 (*C*H₂), 20.7 (*C*H₂).
**IR (neat):** 2860, 1660, 1555, 1465, 1340, 1300, 1250, 1095, 1035, 950, 845, 730 cm-1
**HRMS-ESI:** calcd. for C₄₇H₉₆N₂O₂₂S: 1073.6254; found: 1073.6285.

### Preparation of compound 4 (Scheme 1)

EDCI (1.5 equiv, 72 mmol, 11.17 g) and NHS (1.5 equiv, 72 mmol, 8.36 g) were added to a solution of lipoïc acid (1 equiv, 48 mmol, 10 g) in DMF (30 mL). The reaction mixture was stirred 12 hr at rt and concentrated in vacuo.The crude product was dissolved in DCM and washed three times with H₂O and brine. The organic phase was dried (MgSO₄) and concentrated in vacuo. The product was purified by FCC on silica gel (EtOAc/PE 1:1) to afford **4** as a yellow solid (22.42 mmol, 6.79 g, 46 %).
**¹H-NMR (400 MHz, CDCl₃):** *δ* 3.65-3.49 (m, 1 H, C*H*-S), 3.24-3.05 (m, 2H, C*H*₂-S), 2.83 (s, 4H, 2 x C*H*₂-CO), 2.62 (t, *J* = 7.3 Hz, 2H, C*H*₂-CO), 2.53-2.39 (m, 1H, *H*-CH-CH₂-S), 1.98-1.86 (m, 1 H, *H*-CH-CH₂-S), 1.85-1.65 (m, 4H, CH₂-CH-S, C*H*₂-CH₂-CO), 1.63-1.49 (m, 2H, C*H*₂-CH₂-CH₂-CO).
**¹³C NMR (100 MHz, CDCl₃):** *δ* 169.3 (2 x CO), 168.5 (CO), 56.2 (*C*H), 40.3 (*C*H₂), 38.6 (*C*H₂), 34.5 (*C*H₂), 30.9 (*C*H₂), 28.4 (*C*H₂), 25.7 (*C*H₂), 24.5 (*C*H₂).
**IR (neat):** 2940, 1810, 1780, 1460, 1420, 1410, 1375, 1360, 1205, 1070, 900, 880, 810, 730 cm⁻¹.
**HRMS-ESI:** calcd. for C₁₂H₁₇NO₄S₂Na: 326.0497; found: 326.0497.

### Preparation of compound 5a (Scheme 1)

To a solution of compounds **1a** (1 equiv, 4.72 mmol, 5 g) and **4** (2 equiv, 9.44 mmol, 2.86 g) in DMF (35 mL), Et₃N (1 equiv,4.72 mmol, 477 mg) was added. The reaction mixture was stirred for 48 hr at rt before evaporating the solvent in vacuo. The crude was dissolved in DCM (100 mL) and washed with sat. NH₄Cl (2 x 40 mL). The organic phase was dried (MgSO₄) and concentrated in vacuo. The product was purified by FCC on silica gel (DCM/MeOH 17:1) to afford **5a** as a yellowish amorphous solid (3.71 mmol, 4.63 g, 78 %).
**¹H-NMR (400 MHz, CDCl₃):** *δ* 6.23 (s, 1 H, N*H*), 3.70-3.58 (m, 80 H, 40 x C*H*₂-O-CH₂), 3.65-3.49 (m, 1 H, C*H*-S), 3.56-3.51 (m, 2H, C*H*₂-O), 3.42 (q, *J =* 5.1 Hz, 2H, C*H*₂-NH), 3.37 (t, *J =* 4.2 Hz, 2H, C*H*₂-N₃), 3.23-3.04 (m, 2H, C*H*₂-S), 2.44 (m, 1 H, *H-*CH-CH₂-S), 2.17 (t, *J =* 7.5 Hz, 1 H, C*H*₂-CO), 1.97-1.82 (m, 1 H, *H*-CH-CH₂-S), 1.75-1.59 (m, 4H, CH₂-CH-S, C*H*₂-CH₂-CO), 1.51-1.37 (m, 1H, C*H*₂-CH₂-CH₂-CO).
**¹³C NMR (100 MHz, CDCl₃):** *δ* 172.8 (CO), 77.4 (*C*H₂), 77.1 (*C*H₂), 76.7 (*C*H₂), 70.7 (*C*H₂), 70.6 (*C*H₂), 70.6 (*C*H₂), 70.2 (*C*H₂), 70.0 (*C*H₂), 69.9 (*C*H₂), 56.4 (CH), 50.7 (*C*H₂), 40.2 (*C*H₂), 39.2 (*C*H₂), 38.5 (*C*H₂), 36.3 (*C*H₂), 34.7 (*C*H₂), 28.9 (*C*H₂), 25.4 (*C*H₂).
**IR (neat):** 2870, 2100, 1635, 1555, 1465, 1345, 1280, 1250, 1105, 960, 850 cm⁻¹. **HRMS-ESI:** calcd. for C₅₂H₁₀₂N₄O₂₂S₂Na: 1221.6324; found: 1221.6367.

### Preparation of compound PEG-a II (Scheme 1)

Compound **5a** (1 equiv, 866 µmol, 1 g) was dissolved in dry THF (5 mL) under argon atm. LiAlH₄ (2 equiv, 1.73 mmol, 66 mg) was added portion-wise and the reaction was stirred for 40 min at rt. The reaction was quenched with addition of EtOAc (2 mL) drop-wise then H₂O and the product was extracted with DCM (3 x 30 mL). The combined organic layers were dried (MgSO₄) and concentrated in vacuo to afford **PEG-a II** as a white amorphous solid (791 µmol, 895 g, 91 %).
**IR (neat):** 2885, 1640, 1555, 1465, 1360, 1345, 1280, 1240, 1105, 960, 845 cm⁻¹. **HRMS-ESI:** calcd. for C₅₂H₁₀₆N₂O₂₂S₂: 1175.6757; found: 1175.6735. calcd for: C₅₂H₁₀₄N₂O₂₂S₂Na: 1173.6600; found: 1173.6677.

### Preparation of compound 6 (Scheme 1)

Pyridine (3 equiv, 282.6 mmol, 22.3 g, 22.8 mL) was added dropwise to a mixture of 3-mercaptopropionic acid (1 equiv, 94.2 mmol, 10 g, 8.2 mL) and acetic anhydride (3 equiv, 282.6 mmol, 28.8 g, 26.5 mL). The reaction solution was stirred for 16 hr at rt and concentrated in vacuo. The crude product was dissolved in DCM and washed three times with KHSO₄ 1 M. The organic phase was dried (MgSO₄), concentrated in vacuo and the product was purified by FCC on silica gel (DCM/MeOH 10:1) to afford the desired intermediate as a yellowish oil (53 mmol, 7.85 g, 56 %). EDCI (1.5 equiv, 79.4 mmol, 15.2 g) and NHS (2 equiv, 106 mmol, 12.2 g) were added to a solution of intermediate (1 equiv, 53 mmol, 7. 85 g) in DMF (50 mL). The reaction mixture was stirred 16 hr at rt and concentrated in vacuo.The crude product was dissolved in DCM and washed three times with H₂O and brine. The organic phase was dried (MgSO₄), concentrated in vacuo. The product was purified by FCC on silica gel (EtOAc/PE 7:8) to afford the desired NHS ester as a white solid (37.3 mmol, 9.14 g, 70 %). The NHS ester (1 equiv, 16.3 mmol, 4 g) was dissolved in DCM (15 mL) and Et₃N (1.2 equiv, 19.6 mmol, 1.98 g, 2.73 mL) and propargylamine was added drop-wise (1 equiv, 16.3 mmol, 98 mg, 1.1 mL). The reaction solution was stirred for 16 hr at rt and the DCM layer was subsequently washed with H₂O (2 x 20 mL), sat. NaHCO₃ (2 x 20 mL) and 0.6 M HCl (2 x 20 mL). The organic phase was dried (MgSO₄), concentrated in vacuo. The product was purified by FCC on silica gel (EtOAc/PE 1:1) to afford **6** as a white solid (10.54 mmol, 1.95 g, 63 %).
**¹H-NMR (400 MHz, CDCl₃):** *δ* 6.00 (s, 1 H, N*H*), 4.04 (dd, ³*J* = 5.2, ⁴*J* = 2.6 Hz, 2H, C*H*₂-NH), 3.12 (t, ³*J* = 7.0 Hz, 2H, C*H*₂-S), 2.50 (t, ³*J* = 7.0 Hz, 2H, C*H*₂-CO), 2.32 (s, 3H, C*H*₃), 2.23 (t, ⁴*J* = 2.6 Hz, 1 H, *H*CC).
**¹³C-NMR (100 MHz, CDCl₃):** *δ* 196.3 (CO), 170.4 (CO), 79.5 (Cq), 71.8 (CH), 36.1 (*C*H₂), 30.7 (*C*H₃), 29.3 (*C*H₂), 24.8 (*C*H₂).
**IR (neat):** 3260, 3060, 2920, 1740, 1680, 1660, 1645, 1540, 1420, 1355, 1255, 1210, 1140, 1115, 965 cm⁻¹.
**HRMS-ESI:** calcd. for C₈H₁₁NO₂SNa: 208.0408; found: 208.0409.

### Preparation of compound 7a (Scheme 1)

Compounds **1a** (1 equiv, 1.33 mmol, 1.5 g) and **6** (1.15 equiv, 1.53 mmol, 283 mg) were dissolved in a mixture of DMF: H₂O 1:1 (20 mL). Catalytic amount of CuSO₄ (1 mg) and sodium ascorbate (1 mg) were added and the reaction mixture was stirred for 2 hr at rt. DCM (50 mL) was added and the organic phase was washed with H₂O (20 mL) and 1 % aqueous NH₃ (2 x 20 mL). The organic phase was dried (MgSO₄), concentrated in vacuo. The product was purified by FCC on silica gel (DCM/MeOH 11:1 to 4 :1) to afford **7a** as a yellowish oil (1.09 mmol, 1.44 g, 83 %).
**¹H-NMR (400 MHz, CDCl₃):** *δ* 7.71 (s, 1 H, C*H*), 6.53 (s, 1 H, N*H*), 5.05 (s, 1 H, N*H*), 4.49 (m, 4 H, CH₂-C*H*₂-N, CC*H*₂-NH), 3.85 (m, 2 H, C*H*₂-CH₂-N), 3.68-3.55 (m, 80 H, 40 x C*H*₂-O-CH₂), 3.51 (t, ³*J* = 5.1 Hz, 2 H, C*H*₂-CH₂-NH), 3.28 (m, 2H, C*H*₂-NH), 3.11 (t, ³*J* = 7.0 Hz, 2H, C*H*₂-S), 2.48 (t, ³*J* = 7.0 Hz, 2 H, C*H*₂-CO), 2.29 (s, 3 H, C*H*₃), 1.42 (s, 9H, 3 x C*H*₃).
**¹³C-NMR (100 MHz, CDCl₃):** *δ* 185.9 (*C*O), 170.7 (*C*O), 156.1 (CO), 144.5 (*Cq*), 123.4 (*C*H), 79.2 (*Cq*), 70.6 (C*H*₂), 70.3 (C*H*₂), 69.5 (C*H*₂), 50.4 (C*H*₂), 40.4 (C*H*₂), 36.0 (C*H*₂), 35.2 (C*H*₂), 30.7 (C*H*₃), 28.5 (3 x C*H*₃), 24.9 (*C*H₂).
**IR (neat):** 3335, 2865, 1690, 1730, 1530, 1460, 1350, 1250, 1100, 950, 845 cm⁻¹. **HRMS-ESI:** calcd. for C₅₇H₁₀₉N₅O₂₅SNa: 1318.7030; found: 1318.7072.

### Preparation of compound PEG-a III

4 M HCl in dioxane (13 mL) was added to compound **7a** (1 equiv, 0.97 mmol, 1.3 g) and the reaction mixture was stirred for 2 hr at rt. Dioxane was removed to obtain acylated intermediate as a yellowish oil in a quantitative yield (0.97 mmol, 1.23 mg). **¹H-NMR (400 MHz, CDCl₃):** *δ* 7.92 (s, 2 H, N*H*₂), 7.77 (s, 1 H CC*H*-N), 6.94 (s, 1 H, N*H*), 4.48 (m, 4 H, CH₂-C*H*₂-N, CC*H*₂-NH), 3.84 (m, 4 H, C*H*₂CH₂-N, C*H*₂-NH₂), 3.73-3.51 (m, 80 H C*H*₂-O-CH₂), 3.15 (m, 2 H, C*H*₂-CH₂-NH₂), 3.09 (t, ³*J* = 7.0 Hz, 2 H, C*H*₂-S), 2.48 (t, ³*J* = 7.0 Hz, 2 H, C*H*₂-CO), 2.27 (s, 3 H, CH₃).
**¹³C NMR (100 MHz, CDCl₃):** *δ* 195.9 (CO), 170.9 (*C*O), 144.3 (*Cq*), 123.7 (*C*H), 77.4 (*C*H₂), 70.8 -69.6 (*C*H₂), 69.4 (*C*H₂), 66.8 (*C*H₂), 50.5 (*C*H₂), 40.4 (*C*H₂), 40.1 (*C*H₂), 35.8 (*C*H₂), 34.9 (*C*H₂), 30.6 (*C*H₃), 24.8 (*C*H₂), 20.5 (*C*H₂).
**IR (neat):** 2870, 1670, 1540, 1460, 1350, 1300, 1250, 1100, 950, 845, 730 cm⁻¹. **HRMS-ESI:** calcd. for C₅₂H₁₀₁NsO₂₃S: 1196.6686; found: 1196.6713.

10 % HCl solution was added to acylated intermediate (1 equiv, 836 µmol, 420 mg) and the reaction was stirred for 5 hr at 30 °C. The reaction mixture was dried in vacuo to afford **PEG-a III** as yellowish oil (736 µmol, 850 mg, 88%).
**¹H-NMR (400 MHz, CDCl₃):** *δ* 7.92 (s, 3 H, CC*H*-N, N*H*₂), 7.08 (s, 1 H, N*H*), 4.54 (m, 4 H, C*H*₂-N-N=N, CC*H*₂-NHCO), 3.85 (m, 4 H, C*H*₂CH₂-N, C*H*₂-NH₂), 3.63-3.56 (m, 80 H, C*H*₂-O-CH₂), 3.14 (m, 2 H, C*H*₂-CH₂-NH₂), 2.75 (m, 2 H, C*H*₂-SH), 2.51 (t, ³*J* = 6.5 Hz, 2 H, C*H*₂-CO), 1.60 (t, ³*J* = 8.3 Hz, 2H, S*H*).
**¹³C NMR (100 MHz, CDCl₃):** *δ* 171.1 (*C*O), 144.5 (*Cq*), 123.9 (*C*H), 70.8-69.6 (*C*H₂), 69.1 (*C*H₂), 66.7 (*C*H₂), 50.4 (*C*H₂), 40.1 (*C*H₂), 39.8 (*C*H₂), 34.5 (*C*H₂), 20.3 (*C*H₂). **IR (neat):** 3675, 2870, 1670, 1540, 1455, 1350, 1300, 1250, 1100, 950, 845 cm⁻¹. **HRMS-ESI:** calcd. for C₅₀H₉₉N₅O₂₂S: 1154.6581; found: 1154.6603.

### Preparation of TBA-alg (Scheme 2)

### General procedure for TBA-alg (x)

To convert Na-alg into alginic acid, Kelton HV (2 g) was added to a mixture of aqueous HCl (30 mL, 0.6 N) and ethanol (30 mL), and the solution was stirred overnight at 4°C. The resulting solid was separated by vacuum filtration, washed with ethanol and acetone, and dried quickly under vacuum at 40°C. Dried alginic acid was dispersed in water (100 mL), and TBAOH (5 mL, 40% in water) was added. The solution was dialyzed (8 x against distilled H₂O) and finally freeze-dried to afford TBA-alg (a) as a white solid.

### General procedure for TBA-alg (y)

To convert Na-alg into alginic acid, Kelton HV (2 g) was dissolved in distilled H₂O (200 mL). 20 % formic acid aqueous solution (220 mL) was added and the solution was stirred overnight at 0°C. EtOH (200 mL) was added and the resulting solid was separated by vacuum filtration, washed with H₂O:EtOH (1:1, 3 x 200 mL) and EtOH (200 mL) acetone (200 mL), and dried quickly under vacuum at 40°C. Dried alginic acid was dispersed in water (200 mL), and TBAOH (0.5 equiv, 3.4 mL, 40% in water) was added and the solution was stirred until full dispersion.. The solution was directly freeze-dried without dialysis to afford TBA-alg (b) as a white solid.

### Preparation of Alg-PEG derivatives

### General protocol for the functionalization of TBA-Alg (Scheme 2)

**TBA-alg** (1 equiv, 2.39 mmol, 1 g) was dissolved in DMSO (200 mL) and the solution was stirred for 12 hours to ensure high homogeneity. CDI (1 equiv, 2.39 mmol, 387 mg) previously dissolved in a minimum volume of DMSO (1 mL) was added to the solution and the reaction was stirred at room temperature (rt) for 30 min. Acetone (400 mL) was added to the reaction mixture and the resulting precipitate was filtered and washed with acetone (3 x 100 mL). The precipitate was transferred in a round-bottom flask and distilled water was added (100 mL). After dissolution, amino-PEG derivative (0.2 equiv, 479 µmol) dissolved in a minimum volume of water (1 mL) was added and the reaction mixture was stirred for 2 hr. The reaction was quenched by addition of a 0.05 M NaOH solution (50 mL) and the reaction mixture was directly poured into a dialysis membrane and dialyzed against distilled water. The water dialysis was changed 3 times in one day. Na-citrate solution (0.1 M, 2 mL) was added in the dialysis tube 3 times prior to the dialysis against distilled water. Then TCEP (0.1 M, 2 mL) was added in the dialysis tube 3 times prior to the dialysis against distilled water. The water dialysis was then changed 5 times before adjustment of the pH with addition of 0.05 M NaOH (30 mL) to reach pH 7. The solution was filtered (70 µm) and freeze-dried to obtain the desired product as a white solid.

### Preparation of heterobifunctional PEG derivatives (Scheme 1)

The development of PEG-grafted alg materials for medical applications requires access to a variety of heterocheletic PEG oligomers. Following our previous reports on the production of azido- and amino-silanized PEG molecules [22, 23], straightforward synthetic pathways were developed to produce several thiol-functionalized PEG derivatives. Starting from linear PEG (HOCH₂(CH₂OCH₂)ₙCH₂OH with average n value of 22 or 44, identified as PEG-a or PEG-b) the key intermediates α-amino-ω-azido poly (ethylene glycol) **1a** and **1b,** were obtained in good overall yields (3 steps) (Scheme 1). **PEG I** oligomers were obtained by a coupling reaction with protected 3-mercaptopropanoïc acid **(2),** followed by simultaneous reduction of the azido group and deprotection of the thiol functionality. Alternatively, conjugation to activated lipoic acid followed by reduction in the presence of LiAlH₄ delivered **PEG II** as mixtures of opened (reduced) and closed (oxidized) forms of the lipoyl functionality. Finally, the rigidity of the PEG chain can be decreased by introduction of a triazole moiety using a click reaction catalyzed by copper species to produce **PEG III.**

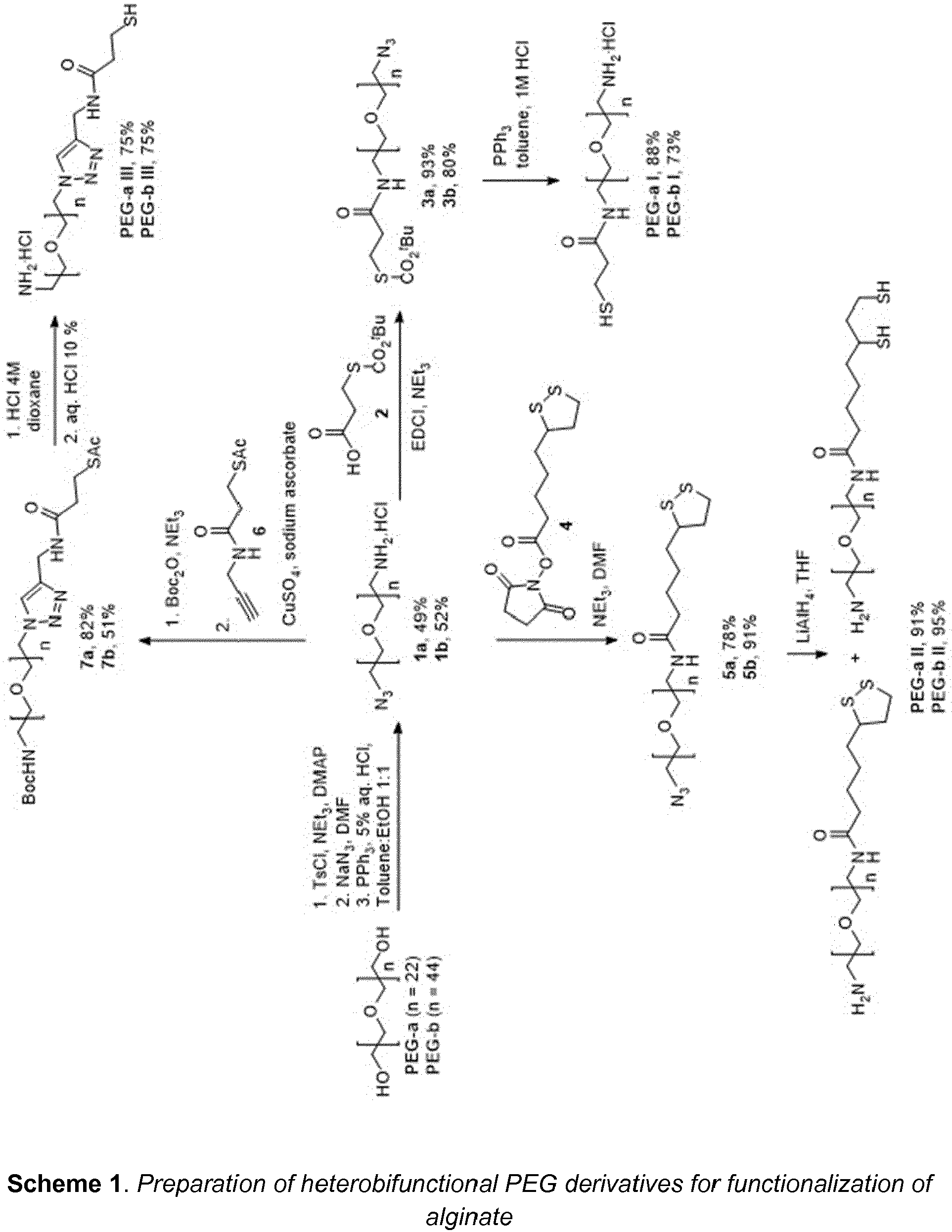

### Functionalization of Na-alg with PEG derivatives (Scheme 2)

In order to maintain all carboxyl groups of Na-alg available for ionic cross-linking, we focused on the modification of hydroxyl groups. Na-alg (Kelton HV) was first converted into TBA-alg to increase solubility in DMSO for further chemical derivatization (Scheme 2). Heterogeneous acidification of Na-alg was performed either in aqueous ethanolic HCl or aqueous formic acid,[24] followed by treatment with TBAOH to afford TBA-alg polymers (**TBA-alg(x); TBA-alg(y)**). Activation of hydroxyl groups in the presence of carbodiimidazole for 0.5 h and subsequent precipitation provided the activated imidazolide. Condensation with PEG derivatives **I-III** followed by purification through dialysis and freeze drying afforded PEG-grafted Na-alg for MS formation. 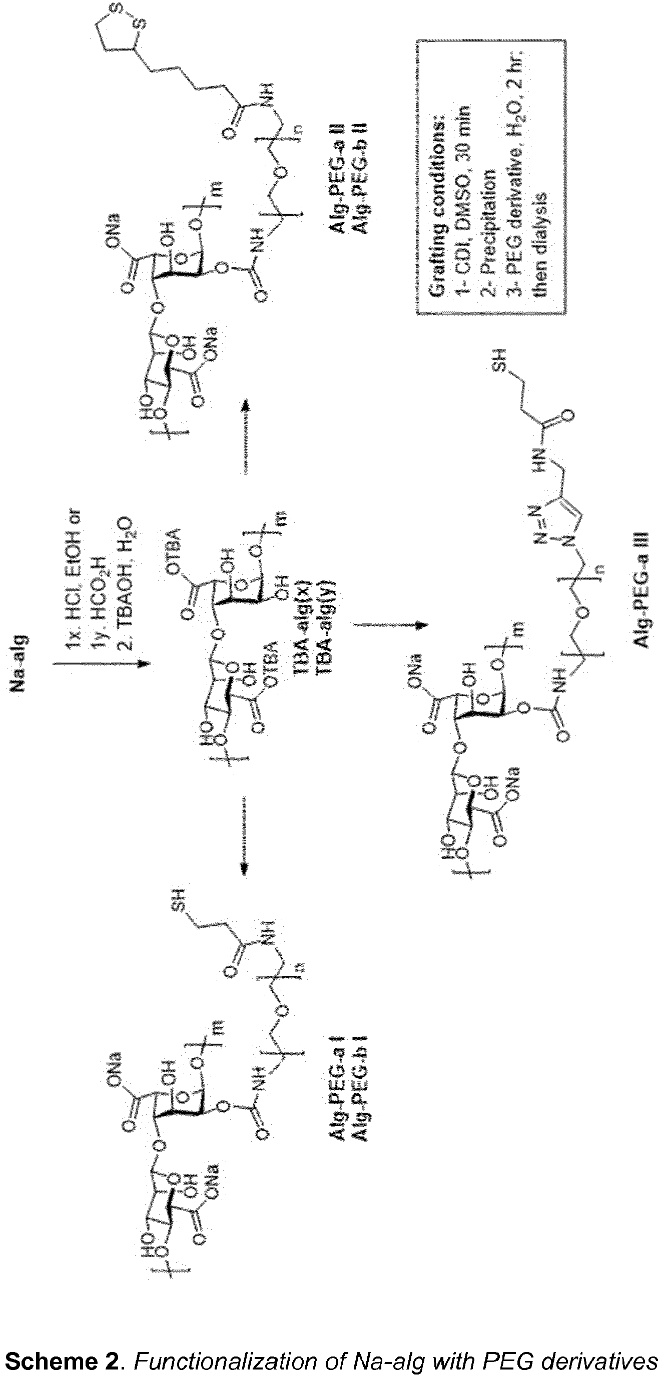 The chemical structures of intermediate **TBA-alg** and PEG grafted alginates were confirmed by NMR analysis. The viscosity of solutions containing various concentrations of the polymers was measured to optimize the conditions for MS formation (Table 1).

**Table 1. Properties of intermediate TBA-alg and PEG grafted alginates**

| **Product** | **Ratio TBA/Alg¹** | **% grafting² (mol %)** | **Viscosity (mPa.s)** | **Formulation N°** |
|---|---|---|---|---|
| TBA-alg(x) | 2.01 | - | 133.1 (2 wt%) | |
| TBA-alg(y) | 2.35 | - | 195.3 (2 wt%) | |
| Alg-PEG-a I(x) | - | 5.9 | 209.0 (3 wt%) | **1** |
| Alg-PEG-a I(y) | - | 4.05 | - | |
| Alg-PEG-b I(x) | - | 13 | 53.4 (4 wt%) | **2** |
| Alg-PEG-a II(x) | - | 7.6 | 160.7 (3 wt%) | **3** |
| Alg-PEG-b II(x) | - | 21 | 160.9 (4 wt%) | **4** |
| Alg-PEG-a III(x) | - | 9.4 | 179.7 (3 wt%) | **5** |

| | | | | |
|---|---|---|---|---|
| ¹Determined by ¹H-NMR; ²Determined by ¹H-NMR; x: initial treatment of Na-alg with ethanolic HCl; y: initial treatment of Na-alg with formic acid. | | | | |

### Microsphere formation from PEG grafted alginate derivatives

Solutions of Alg-PEG, at the concentrations indicated in Table 1, were extruded into a gelation bath containing CaCl₂ as ionic cross-linker to produce one-component MS which were assessed for their size, mechanical resistance to compression, elasticity and permeability. Size and morphology of the MS are reported in Tables 2 and 3.

**Table 2. Size and morphology of MS**

| **Formulation N°** | **1** | **2** | **3** | **4** | **5** |
|---|---|---|---|---|---|
| **Product¹** | **Alg**-**PEG a I** | **Alg-PEG b I** | **Alg-PEG a II** | **Alg-PEG b II** | **Alg-PEG-a III** |
| **Diameter at day 1 (µm)²** | 934.00 ± 100.81 | 1335.90 ± 118.27 | 667.23 ± 98.44 | 715.40 ± 75.72 | 454.80 ± 44.32 |
| **Diameter at day 7 (µm)²** | 913.07 ± 105.56 | 1263.33 ± 121.72 | 642.13 ± 84.52 | 759.70 ± 82.85 | 452.80 ± 42.55 |

| | | | | | |
|---|---|---|---|---|---|
| ¹Grafting with PEG derivatives was performed on TBA-alg(x); ²MS were kept in MOPS at 25°C (10 mM, pH = 7.4); ³Olympus AX70 microscope equipped with an Olympus DP70 color digital camera | | | | | |

For each PEG derivative, an increase of the length of the grafted chain resulted in an increase of the diameter of the beads (formulations **1** vs **2; 3** vs **4**). Evaluation of the MS diameters, after beads formation (day 1) and after one week (day 7), did not show significant changes of the MS.

**Table 3. Size and morphology of MS from Alg-PEG-a / prepared from TBA-alg(y)**

| **Product** | **Diameter at day 3 (µm)²** | **Diameter at day 7 (µm)²** |
|---|---|---|
| **Alg-PEG-a I¹** | 757.53 ± 73.47 | 737.8 ± 79.03 |

| | | |
|---|---|---|
| ¹Grafting with PEG derivatives was performed on TBA-alg(y); ²MS were kept in MOPS at 25°C (10 mM, pH = 7.4); ³Olympus AX70 microscope equipped with an Olympus DP70 color digital camera | | |

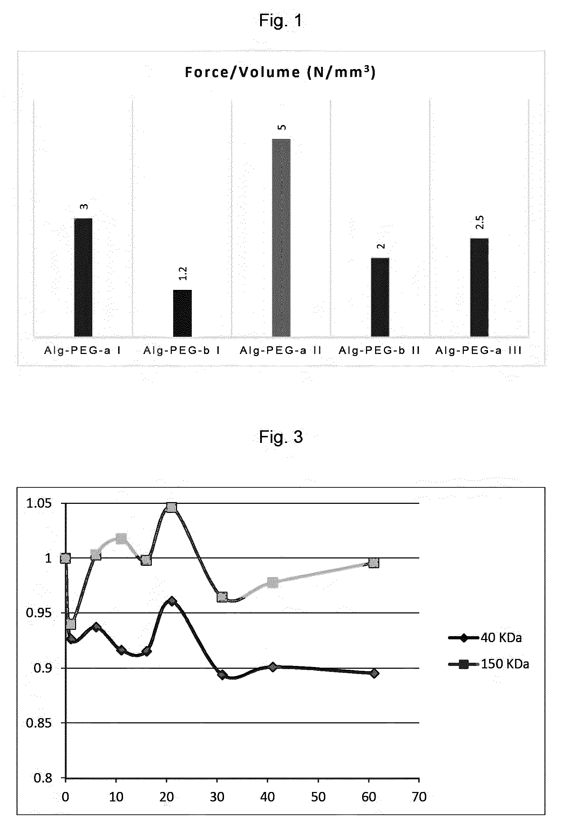

The MS were then evaluated for their mechanical resistance to compression and permeability. Mechanical resistance to uniaxial compression was measured after completion of covalent cross-linking. Upon compression of 40-50% of their initial diameter, all MS showed minimal mechanical resistance. An increase of mechanical resistance was observed from 70% and the resistance became exponential until 90% compression. Figure 1 presents the mechanical resistance to 90% compression of one-component MS listed in Tables 2 and 3.

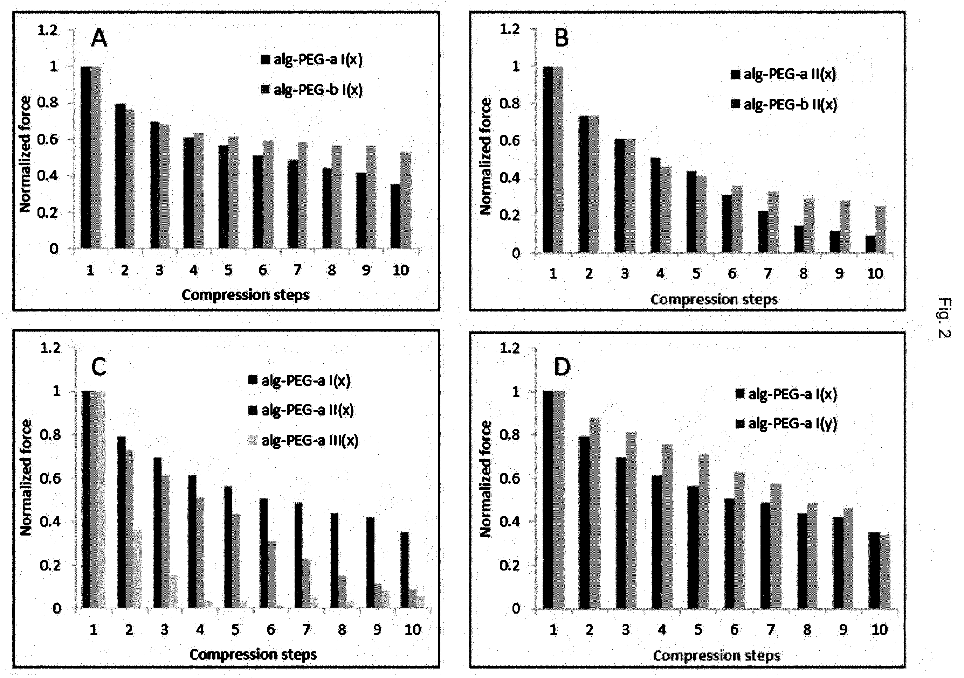

The mechanical properties of the MS were further assessed by evaluation of shape recovery performance upon repeated compression at 90% of the initial MS diameter (Figure 2). One of the shortcomings of the physical properties of Ca-alg MS is their poor elasticity. MS formed by Alg-PEG I and Alg-PEG II showed a good recovery performance.

For the permeability assessment, a MWCO of 150 kg mol⁻¹ is the threshold above which MS intended for cell microencapsulation and further transplantation should exclude any compound. This assessment was performed by ingress diffusion of FITC-dextran standards at 40 kg mol⁻¹ and 150 kg mol⁻¹. Interestingly, all MS formed by PEG grafted alginate derivatives allowed diffusion of 40 kg mol⁻¹ FITC-dextran but excluded 150 kg mol⁻¹ FITC-dextran, indicating that their MWCO is suitable for cell microencapsulation application. This permselectivity requirement is hardly achieved by Ca-alg MS. Figure 3 gives a representative example of the assessment of permeability by ingress diffusion of FITC-dextrans for system Alg-PEG-a I.

### Evaluation of PEG grafted alginate based hydrogels for cell microencapsulation

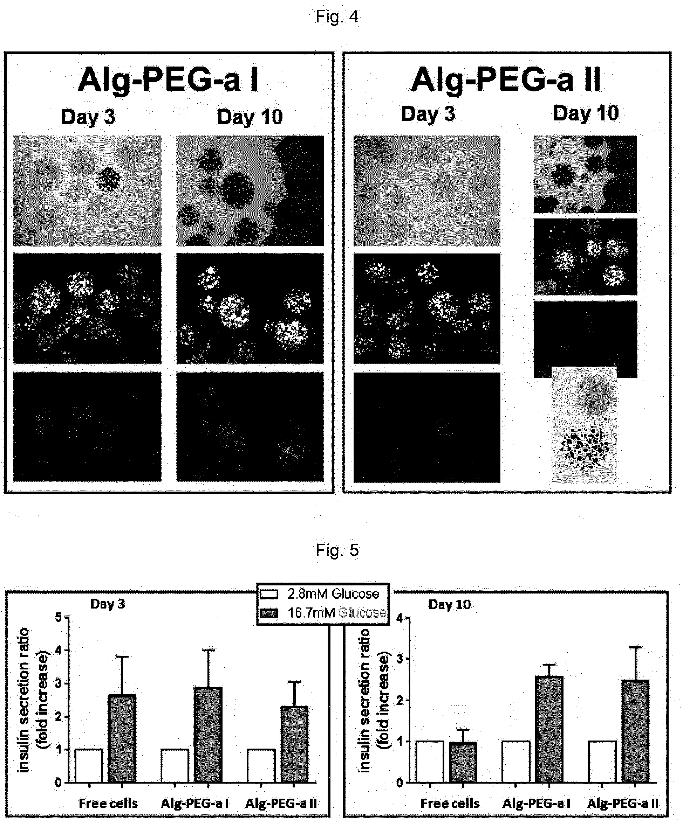

The feasibility of cell microencapsulation in MS formed from Alg-PEG-a I and Alg-PEG-a II was evaluated with cells of the mouse insulinoma cell line MIN6 (Figure 4). The cells were homogeneously distributed within both MS types.

Cell viability was confirmed over 10 days after microencapsulation. The integrity of the MS from Alg-PEG a I system was confirmed as no free cells were identified in the culture medium. The integrity of the MS from Alg-PEG-a II system degraded over time and out-diffusion of the cells was observed around day 10. In light microscopy the microspheres showed a lucent appearance. Stability of the MS can thus be tuned by the chemical composition of the PEG-Alg hydrogel.

Free non-encapsulated MIN6 cells and microencapsulated MIN6 cells were subjected to a glucose-stimulated insulin release assay under static conditions, for both MS at day 3 and day 6 after microencapsulation. Stimulation was done at a glucose concentration of 16.7M, and the fold increase in insulin concentration was calculated with respect to glucose at basal concentration of 2.8M, with correction for the total level of insulin during the culture (Figure 5). The assay outcome was the same for free MIN6 cells and microencapsulated MIN6 cells, using either Alg-PEG-a I or Alg-PEG-a II MS types. The functionalized alginate based hydrogels are thus suitable for maintaining insulin-secreting capacity of microencapsulated cells.

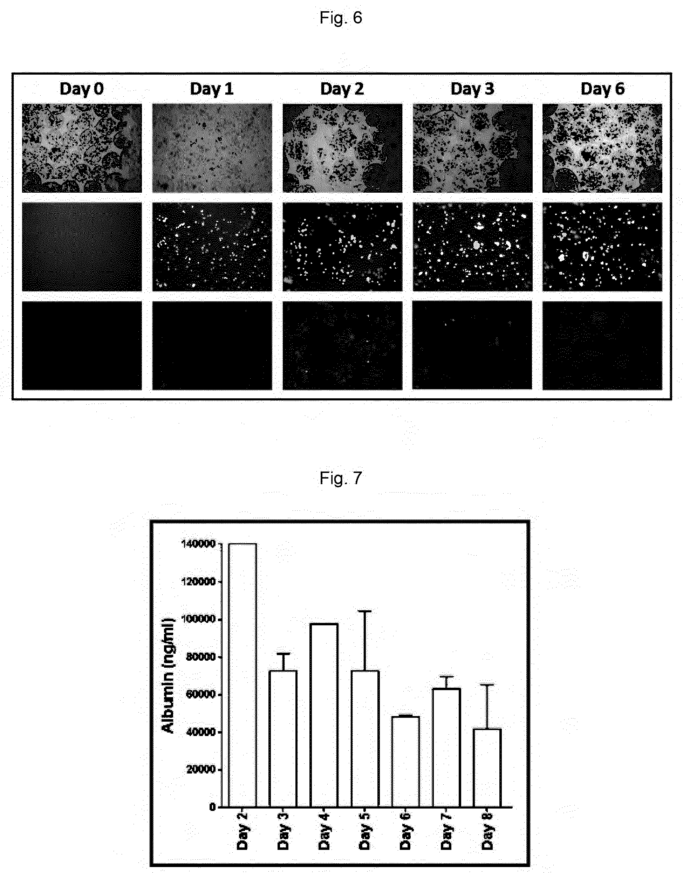

The Alg-PEG-a I system was further evaluated for the microencapsulation of primary porcine hepatocytes. Hepatocytes were isolated from pigs of 10 kg. The microencapsulated primary hepatocytes remained viable for a culture period up to 10 days (Figure 6). This is illustrated in Figure 6 for a culture till 6 days.

The production of albumin by microencapsulated hepatocytes was measured daily, with daily exchange of the medium (Figure 7). The capacity for albumin secretion is maintained upon microencapsulation, with a tendency to decrease in time.

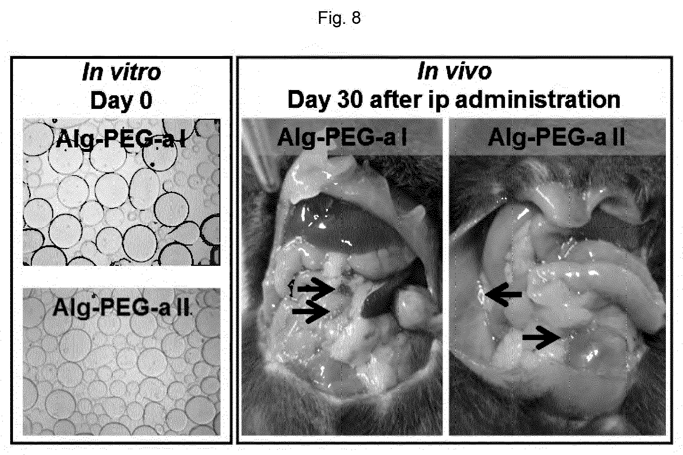

Finally, the compatibility of Alg-PEG-a I and Alg-PEG-a II for cell transplantation was assessed by the transplantation of empty MS formed from both systems in immune-competent mice, with a follow-up period of 30 days. Then, the MS were macroscopically inspected and retrieved (Figure 8). At macroscopic inspection MS were visible. There were no signs of inflammation, connective tissue formation nor fibrosis.

### REFERENCES

1. Lee, K. Y.; Mooney, D. J. Alginate: Properties and biomedical applications. Prog. Polym. Sci. 2012, 37, 106-126.
2. Goh, C. H.; Heng, P. W. S.; Chan, L. W. Alginates as a useful natural polymer for microencapsulation and therapeutic applications. Carbohydr. Polym. 2012, 88, 1-12.
3. Nedovic, V.; Willaert, R. Applications of Cell Immobilization Biotechnology; Springer: Dordrecht, 2005.
4. O'Sullivan, E. S.; Vegas, A.; Anderson, D. G.; Weir, G. C. Islets transplanted in immunoisolation devices: A review of the progress and the challenges that remain. Endocr. Rev. 2011, 32, 827-844.
5. Basta, G.; Calafiore, R. Immunoisolation of pancreatic islet grafts with no recipient's immunosuppression: Actual and future perspectives. Curr. Diabetes Rep. 2011, 11, 384-391.
6. Drury, J. L.; Dennis, R. G.; Mooney, D. J. The tensile properties of alginate hydrogels. Biomaterials 2004, 25, 3187-3199.
7. Moya, M. L.; Morley, M.; Khanna, O.; Opara, E. C.; Brey, E. M. Stability of alginate microbead properties in vitro. J. Mater. Sci.: Mater. Med. 2012, 23, 903-912.
8. King, A.; Strand, B.; Rokstad, A. M.; Kulseng, B.; Andersson, A.; Skjåk-Bræk, G.; Sandler, S. Improvement of the biocompatibility of alginate/poly-L-lysine/alginate microcapsules by the use of epimerized alginate as a coating. J. Biomed. Mater. Res., Part A 2003, 64, 533-539.
9. Chen, A. Z.; Bai, Y.; Wang, S. B.; Liu, Y. G.; Chen, Z. X. Molecular biocompatibility evaluation of poly-L-ornithine-coated alginate microcapsules by investigating mRNA expression of proinflammatory cytokines. J. Biomimetics Biomater. Tissue Eng. 2012, 14, 53-64.
10. Wandrey, C.; Espinosa, D.; Rehor, A.; Hunkeler, D. Influence of alginate characteristics on the properties of multi-component microcapsules. J. Microencapsulation 2003, 20, 597-611.
11. Hu, X.; Li, D.; Gao, C. Chemically crosslinked chitosan hydrogel loaded with gelatin for chondrocyte encapsulation. Biotechnol. J. 2011, 6, 1388-1396.
12. Fu, Y.; Xu, K.; Zheng, X.; Giacomin, A. J.; Mix, A. W.; Kao, W. J. 3D cell entrapment in crosslinked thiolated gelatin-poly(ethylene glycol) diacrylate hydrogels. Biomaterials 2012, 33, 48-58.
13. Brunsen, A.; Ritz, U.; Mateescu, A.; Höfer, I.; Frank, P.; Menges, B.; Hofmann, A.; Rommens, P. M.; Knoll, W.; Jonas, U. Photocrosslinkable dextran hydrogel films as substrates for osteoblast and endothelial cell growth. *J. Mater. Chem.* **2012,** *22*, 19590-19604.
14. Bian, L.; Hou, C.; Tous, E. The influence of hyaluronic acid hydrogel crosslinking density and macromolecular diffusivity on human MSC chondrogenesis and hypertrophy. Biomaterials 2013, 34, 413-421.
15. Rockstad, A. M.; Brekke, O. L.; Steinkjer, B.; Ryan, L.; Kolláriková, G.; Strand, B. L.; Skjåk-Braek, G.; Lambris, J. D.; Lacík, I.; Mollnes, T. E.; Espevik, T. The induction of cytokines by polycation containing microspheres by a complement dependent mechanism. Biomaterials 2013, 34, 621-630.
16. Vériter, S.; Mergen, J.; Goebbels, R.-M.; Aouassar, N.; Grégoire, C.; Jordan, B.; Levêque, P.; Gallez, B.; Gianello, P.; Dufrane, D. in vivo selection of biocompatible alginates for islet encapsulation and subcutaneous transplantation. Tissue Eng.: PartA, 2010, 16, 1503-1513.
17. Dang, T. T.; Thai, A. V.; Cohen, J.; Slosberg, J. E.; Siniakowicz, K.; Doloff, J. C.; Ma, M.; Hollister-Lock, J.; Tang, K. M.; Gu, Z.; Cheng, H.; Weir, G. C.; Langer, R.; Anderson, D. G. Enhanced function of immuno-isolated islets in diabetes therapy by co-encapsulation with an anti-inflammatory drug. Biomaterials 2013, 34, 5792-5801.
18. Mahou, R.; Wandrey, C. Alginate-poly(ethylene glycol) hybrid microspheres with adjustable physical properties. Macromolecules 2010, 43, 1371-1378.
19. Mahou, R.; Kolláriková, G.; Gonelle-Gispert, C.; Meier, R. P. H.; Schmitt, F.; Tran, N. M.; Dufresne, M.; Altimar, I.; Lacík, I.; Bühler, L.; Juillerat-Jeanneret, L; Legallais, C.; Wandrey, C. Combined electrostatic and covalent polymer network for cell microencapsulation. Macromol. Symp. 2013, 329, 49-57.
20. Mahou, R.; Kolláriková, G.; Gonelle-Gispert, C.; Meier, R. P. H.; Schmitt, F.; tran, N. M.; Dufresne, M.; Altimar, I.; Lacík, I.; Bühler, L.; Juillerat-Jeanneret, L; Legallais, C.; Wandrey, C. Combined electrostatic and covalent polymer network for cell microencapsulation. Macromol. Symp. 2013, 329, 49-57.
21. Mahou, R.; Borcard, F.; Crivelli, V.; Montanari, E.; Passemard, S.; Noverraz, F.; Gerber-Lemaire, S.; Bühler, L.; Wandrey, C. Tuning the Properties of Hydrogel Microspheres by Adding Chemical Crosslinking Functionality to Sodium Alginate. Chem. Mater. 2015, 27, 4380-4389.
22. Mahou, R.; Wandrey, C. Versatile Route to Synthesize Heterobifunctional Poly(ethylene glycol) of Variable Functionality for Subsequent Pegylation. Polymers 2012, 4, 561-589.
23. Passemard, S.; Staedler, D.; 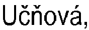 , L.; Schneiter, G. S.; Kong, P.; Bonacina, L.; Juillerat-Jeanneret, L.; Gerber-Lemaire, S. Convenient synthesis of bifunctional azido and amino-silanized poly(ethylene glycol) suitable for the functionalization of iron oxide nanoparticles for biomedical applications. *Bioorg. Med. Chem. Lett.* **2013,** *23*, 5006-5010.
24. Schleeh, T.; Madau, M.; Roessner, D. Synthesis enhancements for generating highly soluble tetrabutylammonium alginates in organic solvents. Carbohydr. Polym. 2014, 114, 493-499.

## Claims

1. A functionalized hydrogel based on polysaccharide grafted with at least one moiety wherein the hydrogel is an anionic polysaccharide and the moiety is grafted on at least one hydroxyl group of said anionic polysaccharide.

2. The functionalized hydrogel of claim 1, wherein the anionic polysaccharide comprises one or more carboxylic acid functional groups.

3. The functionalized hydrogel of any one of the preceding claims, wherein the anionic polysaccharide is selected from the group comprising dextran, alginate, hyaluronan and galacturonan, or a salt thereof.

4. The functionalized hydrogel of any one of the preceding claims, wherein the moiety is grafted on hydroxyl groups of said anionic polysaccharide through a carbamate bond, with a degree of grafting comprised between 5 and 30 mol%.

5. The functionalized hydrogel of any one of the preceding claims, wherein the moiety is selected from the group comprising PEG and a therapeutic molecule.

6. The functionalized hydrogel of claim 5, wherein the PEG, or a derivative thereof, is a heterobifunctional PEG of formula I, II and/or III 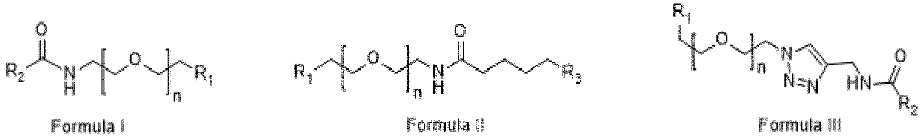 wherein
n = 8 to 50;
R₁ is independently selected from NH₂, N₃, OH, C₁₋₆ alkyl-CO₂H;
R₂ is independently selected from (CH₂)ₚSH, with p= 2 to 10, 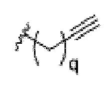 with q = 2 to 5, 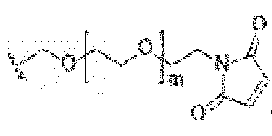 with m= 2 to 10;
R₃ is independently selected from

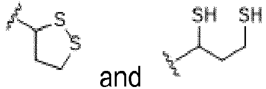

7. The functionalized hydrogel of any one of the preceding claims, wherein the hydrogel is in the form of a microsphere.

8. Composition comprising a functionalized hydrogel of any one of claims 1 to 7 combined with at least one second element selected among cells, proteins, nucleic acids or other molecules.

9. Pharmaceutical composition comprising a composition of claim 8 and a pharmaceutically acceptable carrier.

10. A process for preparing a functionalized hydrogel, the process comprising reacting an anionic polysaccharide with a moiety functionalized to allow the formation of a covalent bond between the polysaccharide and the moiety on at least one hydroxyl group of the polysaccharide.

11. The process of claim 10, wherein the anionic polysaccharide comprises one or more carboxylic acid functional groups.

12. The process of any one of claims 10 to 11, wherein the anionic polysaccharide is selected from the group comprising dextran, alginate, hyaluronan and galacturonan, or a salt thereof.

13. The process of any one of claims 10 to 12, wherein the functionalized moiety is a heterobifunctional PEG, or a derivative thereof, of formula I, II and/or III: 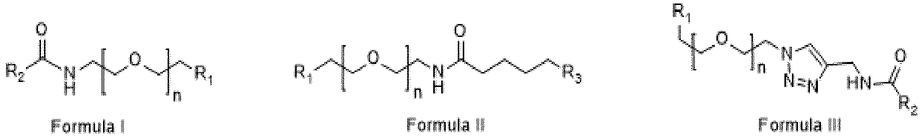 wherein
n = 8 to 50;
R₁ is independently selected from NH₂, N₃, OH, C₁₋₆ alkyl-CO₂H;
R₂ is independently selected from (CH₂)ₚSH, with p= 2 to 10, 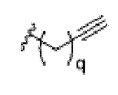 with q = 2 to 5, 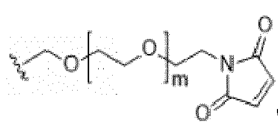 with m= 2 to 10;
R₃ is independently selected from

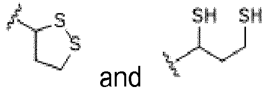

14. A process for preparing a functionalized alginate based hydrogel, the process comprising
i) converting one or more carboxylic acid functional groups of alginate or one or more carboxylate functional groups of alginate salt into TBA carboxylates,
ii) modifying one or more hydroxyl groups of said alginate, or salt thereof, by reacting one or more hydroxyl groups in the presence of carbodiimidazole followed by precipitation,
iii) preparing thiol-functionalized PEG derivatives (PEG I, PEG II and PEG III) by
iii1) reacting the intermediates α-amino-ω-azido poly (ethylene glycol) with protected 3-mercaptopropanoïc acid, followed by simultaneous reduction of the azido group and deprotection of the thiol functionality,
or,
iii2) conjugating intermediates α-amino-ω-azido poly (ethylene glycol) to activated lipoic acid followed by reduction in the presence of LiAlH₄ so as to deliver PEG II as mixtures of opened (reduced) and closed (oxidized) forms of the lipoyl functionality,
or
iii3) contacting intermediates α-amino-ω-azido poly (ethylene glycol) 1 a and 1 b with a triazole moiety using a click reaction catalyzed by copper species to produce PEG III,
iv) grafting the modified alginate with PEG I, PEG II and/or PEG III to allow the formation of a covalent bond between the functionalized alginate and PEG I, PEG II and/or PEG III on one hydroxyl group of said alginate or salt thereof,
v) purifying alginate grafted to PEG through dialysis and freeze drying.

15. A heterobifunctional PEG, or a derivative thereof, of formula I, II and/or III: 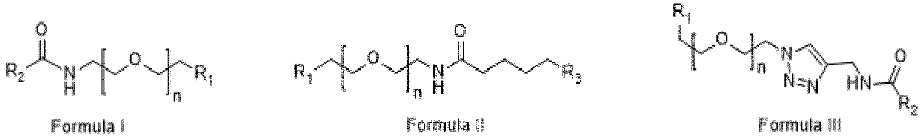 wherein
n = 8 to 50;
R₁ is independently selected from NH₂, N₃, OH, C₁₋₆ alkyl-CO₂H;
R₂ is independently selected from (CH₂)ₚSH, with p= 2 to 10, 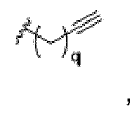 with q = 2 to 5, 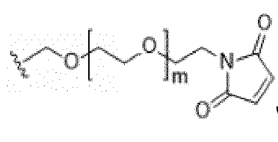 with m= 2 to 10;
R₃ is independently selected from
